(19) Europäisches Patentamt
European Patent Office
Office européen des brevets

(11) Publication number: **0 056 322**
**B1**

(12) # EUROPEAN PATENT SPECIFICATION

(45) Date of publication of patent specification: **21.11.85**

(51) Int. Cl.⁴: **C 07 K 15/06,** A 61 K 39/395, A 61 K 31/475

(21) Application number: **82300116.9**

(22) Date of filing: **11.01.82**

(54) **Immunoglobulin conjugates.**

(30) Priority: **12.01.81 GB 8100847**

(43) Date of publication of application:
**21.07.82 Bulletin 82/29**

(45) Publication of the grant of the patent:
**21.11.85 Bulletin 85/47**

(84) Designated Contracting States:
**AT BE CH DE FR GB IT LI LU NL SE**

(56) References cited:
**EP-A-0 000 938**
**EP-A-0 017 507**
**DE-A-2 456 224**
**FR-A-2 349 335**
**FR-A-2 437 213**
**GB-A-1 446 536**

**CHEMICAL ABSTRACTS, vol. 96, no. 8, February 15, 1982, page 27, 2nd column, Abstract no. 45940e Columbus Ohio (US) R.J. JOHNSON et al.: "A vindesine-anti-CEA conjugate cytotoxic for human cancer cells in vitro"**

(73) Proprietor: **Lilly Industries Limited**
**Lilly House Hanover Square**
**London W1R 0PA (GB)**

(72) Inventor: **Rowland, George Ferdinand**
**55, Dukes Wood Drive**
**Gerrards Cross Buckinghamshire (GB)**
Inventor: **Simmonds, Robin George**
**36, Oaklands Drive**
**Wokingham Berkshire (GB)**

(74) Representative: **Hudson, Christopher Mark et al**
**Erl Wood Manor**
**Windlesham Surrey GU20 6PH (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to novel immunoglobulin conjugates which are anti-cancer agents.

In the chemotherapeutic treatment of cancer it is sometimes necessary to employ drugs that are limited in their effectiveness by unwanted side effects on the patient and efforts are continually being made to minimise such efects. One such drug is vindesine, a compound of the following formula (I)

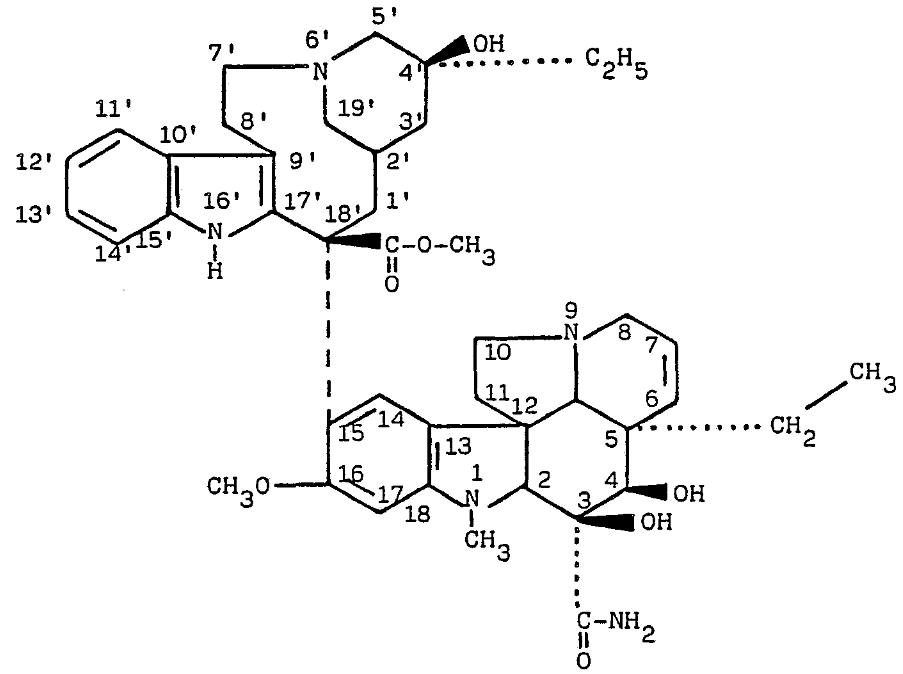

(I)

Vindesine is disclosed, for example, in British Patent 1,463,575 which describes its preparation and biological acitivity.

This invention provides a novel conjugate comprising an immunoglobulin or immunoglobulin fragment modified by one or more groups of the following formula which are covalently linked to it:

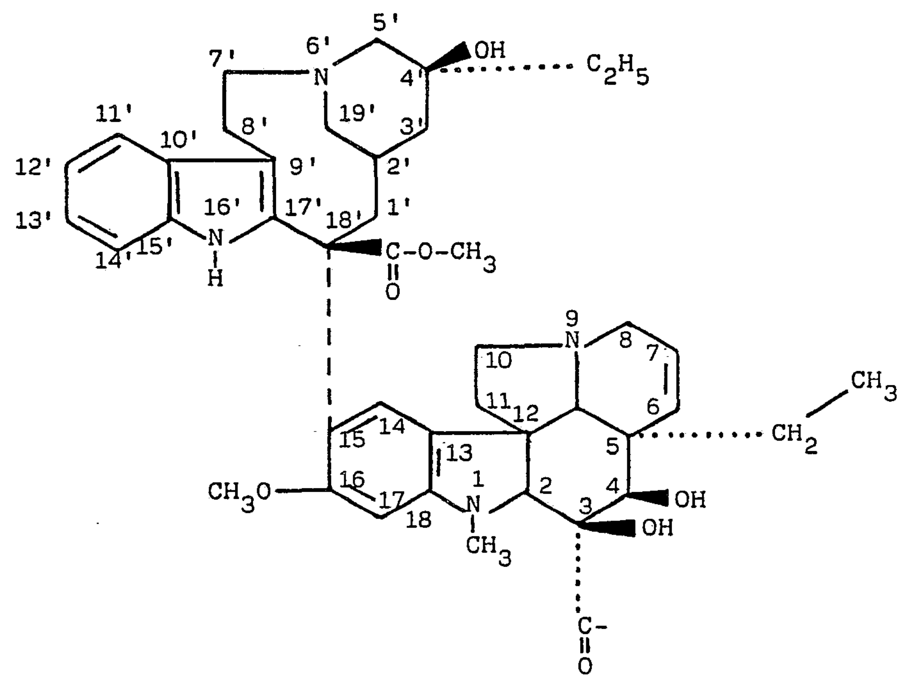

(II)

The immunoglobulin or fragment is an antibody or a fragment of an antibody with antigen recognising properties. The preferred immunoglobulin material is an antibody or a fragment of an antibody adapted for

recognition of antigens on the surface of malignant cells of the type occurring in the human body. However immunoglobulin materials of other kinds are also included within the scope of the invention since they may be of use in treatment of animals and in control of assay experiments.

The novel conjugates are useful in the treatment of cancers. They are potentially more effective and have fewer side effects by virtue, *inter alia,* of their ability to increase the concentration of the cytotoxic drug at the site of action.

It is also to be understood that the invention includes conjugates for use in an indirect system in which they are used to recognise an antibody specific to the cell surface antigen.

Immunoglobulins specific to antigens on the surface of cells to be killed, and techniques for their production from the serum of immunised aniamls or by culturing hybridomas secreting monoclonal products, are well known. The preferred type of antibody is an immunoglobulin of the IgG class. Some representative immunoglobulins are as follows

(i) Ig from goats or sheep immunised with carcinoembryonic antigen

(ii) Ig from rabbit anti-acute lymphoblastic leukemia serum

(ii) Ig from various primate antisera raised against acute lymphoblastic leukemia, acute myleoblastic leukemia, chronic lymphoblastic leukemia and chronic granulocytic leukemia

(iv) Ig from goats or sheep immunised with lung carcinoma material

(v) monoclonal Ig from mouse hybridomas secreting anti-human colorectal carcinoma antibodies

(vi) monoclonal Ig from mouse hybridomas secreting anti-human melanoma antibodies.

(vii) monoclonal Ig from mouse hybridomas secreting antibodies reacting with human leukemia cells

(viii) monocolonal Ig from mouse hybridomas secreting antibodies reacting with human neuroblastoma cells

(ix) monoclonal Ig from mouse hybridomas secreting antibodies reacting with human breast cancer antigens

(x) monoclonal Ig from mouse hybridomas secreting antibodies reacting with human ovarian carcinoma cells

(xi) monoclonal Ig from mouse hybridomas secreting antibodies reacting with human osteosarcoma cells.

As indicated above, the conjugate can also be made with immunoglobulin fragments, referred to as Fab, Fab' or $F(ab')_2$ or IgM monomer derived from an antibody by, for example, proteolytic enzyme digestion. Such materials and methods of preparation are well known and it may be mentioned that preferred proteolytic enzymes are pepsin and papain.

Vindesine formula (I) can be bound to the immunoglobulin material via the carbon atom of the carboxyl residue at the 3-position by reaction with the appropriate azide derivative and the invention includes, as a further aspect, a process for preparing an anti-cancer agent which comprises reacting an immunoglobulin or immunoglobulin fragment with an azide compound of the formula (III)

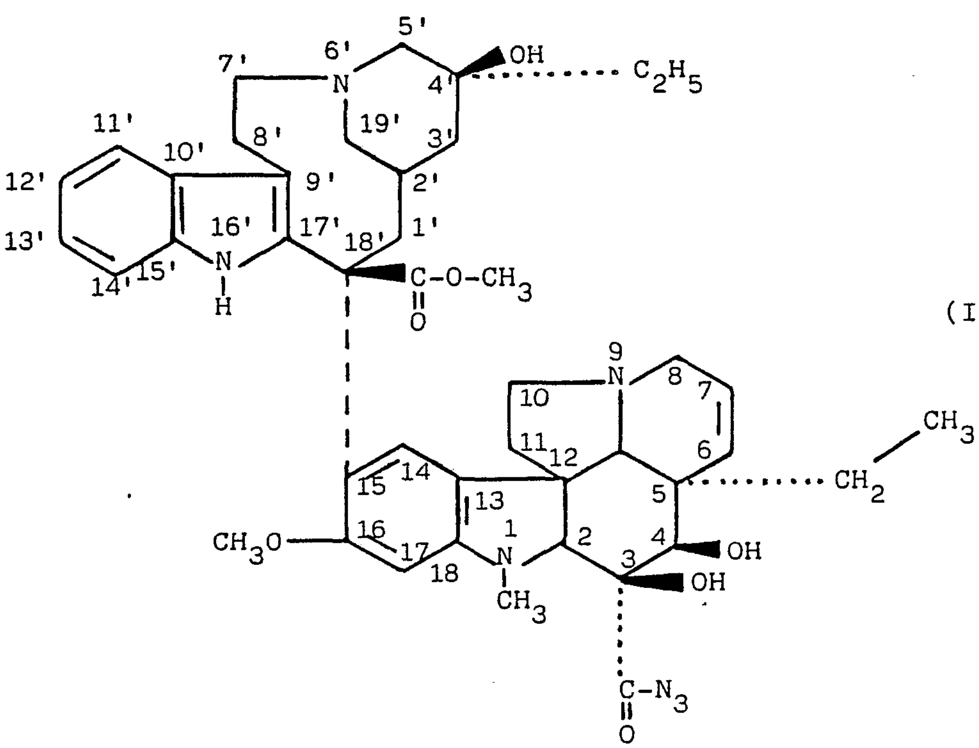

(III)

This process represents a convenient way of linking the drug vindesine to immunoglobulin under mild conditions whilst maintaining the structural integrity of the immunoglobulin.

The process is preferably carried out in an aqueous medium and at a temperature of from 5°C to 250°C, for example at room temperature, and at a pH of 8·5 to 9·5, preferably 9·0, and results in the attachment by covalent linkage of one or more vinca residues at the free amino groups of the immunoglobulin molecule, for example, amimo groups derived from lysine residues. The number of residues attached will depend on the concentration of the reactants and the duration of the reaction but the average number is usually from 1 for example from 2 to 5.

For example in carrying out the reaction, a solution of the azide in a suitable solvent such as dioxan is slowly added dropwise to a buffered solution of immunoglobulin in for example 0·34 *M* borate buffer at pH 9. The conjugate is isolated by gel filtration and stored in saturated ammonium sulphate solution being readily brought back into solution by dialysis with borate buffer, or alternatively it can be stored in a refrigerator at 4°C or frozen at for example −20°C.

The azide reactant of formula (III) can be readily prepared from vinblastine, vinblastine sulphate or desacetylvinblastine by preferential hydrazinolysis of the $C^3$— ester followed by diazotisation as for example described in J. Med. Chem., 1978, Vol. 21, No. 1 pages 88 to 96, and in British Patent 1,463,575 referred to above. Hydrazinolysis of vinblastine at moderate temperatures in methanol solution proceeds yielding predominantly the monohydrazide and reaction of this product at temperatures of 0°C to 10°C with hydrochloric acid and sodium nitrite in a suitable aqueous solvent, yields the desired azide of formula (III) (desacetylvinblastine acid azide).

Alternatively the azide of formula (III) can be prepared at a temperature of below 5°C as described above, and, without isolating the azide, the pH is adjusted to 9 and a water miscible solvent, such as for example dioxan, added. The immunoglobulin borate buffer pH9 is then added and the reaction mixture allowed to rise to room temperature. Excess azide is destroyed with dilute ammonia and the conjugate purified by gel filtration.

Evaluation of the conjugate can be carried out using well known techniques such as affinity chromotography. The efficacy of the conjugate can be estimated by counting the number of viable cells after treatment of a suspension of tumour cells with the conjugate, or from measurements of the uptake of tritiated uridine. Protein and drug concentrations are determined by measuring optical densities of conjugate solutions at two wavelengths, for example 270 and 280 nm, and relating the values obtained to those for the free drug and unconjugated immunoglobulin at the same two wavelengths.

The novel conjugates of the invention are useful in the treatment of cancers and as such are preferably prepared for use in formulations suitable for injection. Thus the invention includes a pharmaceuticl formulation, for example an injectable preparation, comprising a conjugate of the invention together with a pharmaceutically-acceptable carrier or diluent such as are well known in the art. It is preferably in unit dosage form each dosage containing for example from 0.01 to 2 mg of the active ingredient (in terms of the vindesine moiety).

The novel conjugates are effective over a wide dosage range and for example for the treatment of adult humans dosages per week will normally fall within the range of 1 to 10 mg/kg (vindesine moiety) more usually in the range of from 3 to 9 mg/kg. However it will be understood that the amount of compound actually administered will be determined by a physician in the light of the relevant circumstances, including the condition to be treated and the chosen route of administration.

The invention is illustrated by the following Examples

## Example 1

Preparation of vindesine-sheep-anti-carcinoembryonic antigen conjugate

Desacetyl vinblastine acid hydrazide (6 mg) was dissolved in IN HCl (340 µl) and the solution cooled to 4°C. Sixty microlitres of a 10 mg/ml aqueous solution of sodium nitrite were added and the reaction stirred at 4°C. After 5 minutes, 1*N* NaOH (300 µl) and dioxan (120 µl) were added with vigourous stirring, following by 200 µl of a 21.3 mg/ml solution of sheep-anti-CEA in 0.34 *M* borate buffer pH 8.6. The mixture was stirred at room temperature for 2 hours maintaining pH 9 using N/10 NaOH. Excess azide was destroyed by adding 1*N* $NH_4$ OH(60 µl) and stirring for a further hour. The product was isolated by gel filtration on a 1 × 25.5 cm (20 µl) column of Bio-Gel®p-6 equilibrated with phosphate buffered saline. The excluded peak was collected (2.66 ml), and assayed for protein content by the Lowry method and for vindesine by difference spectroscopy. The conjugate so prepared contained 4.6 moles vindesine per mole of Ig.

## Example 2

Preparation of vindesine-mouse-monoclonal anti-carcinoembryonic antigen conjugate

Desacetylvinblastine acid hydrazie (10 mg) was dissolved in 1*N* HCl (0.56 ml) and the solution cooled to 4°C. One hundred microlitres of a 10 mg/ml solution of sodium nitrite were added and the reaction stirred at 4°C. After 5 minutes, 1*N* NaOH (0.5 ml) and dioxan (0.2 ml) were added with vigorous stirring, followed by 0·5 ml of a 15.6 mg/ml solution of mouse monoclonal anti-CEA in 0.34 *N* borate buffer pH 8.6. The mixture was stirred at room temperature for 2 hours maintaining pH 9 using N/10 NaOH. Excess azide was destroyed by adding 1*N* $NH_4OH$ (0.1 ml) and stirring for a further hour. The product was isolated by gel filtration on a 1 × 27 cm (21 ml) column of Bio-Gel®P-6 equilibrated with phosphate buffered saline. The

excluded peak was collected (4.07 ml) and assayed for protein and vindesine by spectroscopy at 270 nm and 280 nm. The conjugate so prepared contained 2.1 moles vindesine per mole of Ig.

Example 3

Preparation of vindesine-rabbit anti-mouse Ig conjugate

Desacetylvinblastine acid hydrazie (12 mg) was dissolved in 1*N* HCl (0.67 ml) and the solution cooled to 4°C. One hundred and twenty microlitres of a 10 mg/ml solution of sodium nitrite were added and the reaction stirred at 4°C. After 5 minutes, 1*N* NaOH (0.6 ml) and dioxan (0.24 ml) were added with vigorous stirring, followed by 1.0 ml of an 11.7 mg/ml solution of rabbit anti-mouse Ig in 0.34 *M* borate buffer pH 8.6. The mixture was stirred at room temperature for 2 hours maintaining pH9 using N/10 NaOH. Excess azide was destroyed by adding 1*N* $NH_{4OH}$ (0.12 ml) and stirring for a further hour. The product was isolated by gel filtration on a 1.5 × 26 cm (46 ml) column of Bio-Gel®P-6 equilibrated with phosphate buffered saline. The excluded peak was collected (6.22 ml) and assayed for protein and vindesine by spectroscopy at 270 nm and 280 nm. The conjugate so prepared contained 3.2 moles vindesine per mole of Ig.

Example 4

A preparation of cells growing in culture was dispensed into microlite trays at a level of $10^4$ cells per well. After allowing the cells 24 hours for establishment, replicate wells were treated with a range of concentrations of a conjugate or a control preparation. After six days in culture, the number of cells present in the wells was assessed by direct counting, using an image analyser linked to an inverted microscope.

In one example, a human colon adenocarcinoma cell line was treated with videsine linked to a monoclonal anti-carcinoembryonic antigen antibody in a molar ratio of 2.1 moles vindesine per mole of Ig (this was the conjugate of Example 2) and the cytostatic effect measured by cell counts, as follows:

| Conjugated drug concentration (vidensine, µg/ml) | Number of cells/well after six days (mean ± standard deviation) 4 replicates | % of control |
|---|---|---|
| 0 | $16.3 \pm 4.8 \times 10^4$ | 100 |
| 0.08 | $13.8 \pm 2.5 \times 10^4$ | 84.6 |
| 0.40 | $12.6 \pm 1.9 \times 10^4$ | 77.4 |
| 2.00 | $10.8 \pm 3.9 \times 10^4$ | 66.7 |
| 10.00 | $1.9 \pm 0.6 \times 10^4$ | 11.9 |

**Claims for the Contacting States: BE CH DE FR GB IT LI LU NL SE**

1. A conjugate comprising an immunoglobulin or immunoglobulin fragment modified by one or more groups of the following formula which are covalently linked to it:

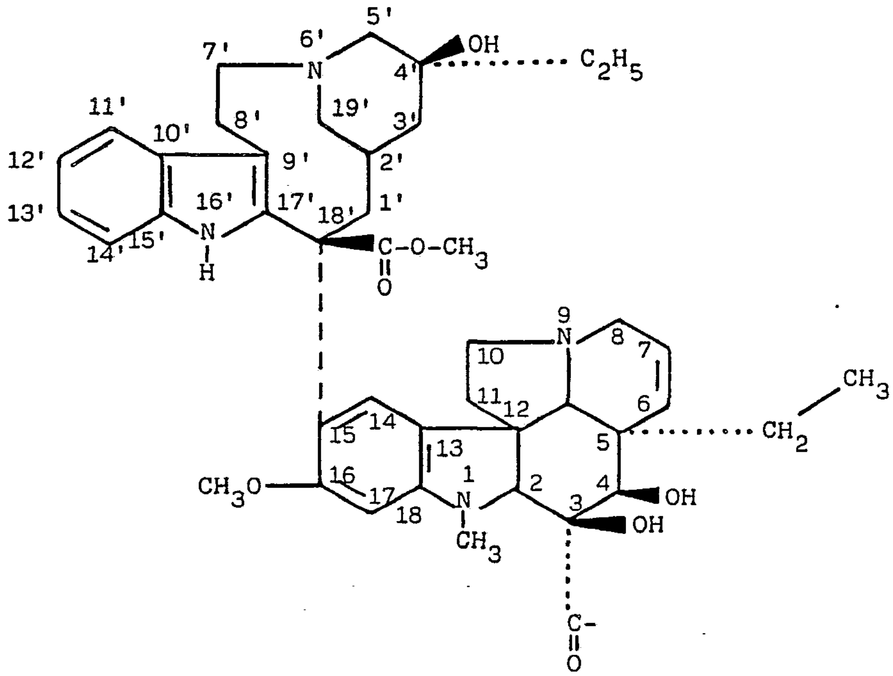

(II)

2. A conjugate according to claim 1 utilising an IgG immunoglobulin.

3. A conjugate according to either of claims 1 and 2 in which the immunoglobulin or immunoglobulin framgent is modified by an average of from 2 to 5 grops of formula (II).

4. A process for preparing a conjugate according to claim 1 which comprises reacting an immunoglobulin or immunoglobulin fragment with an azide compound of the formula

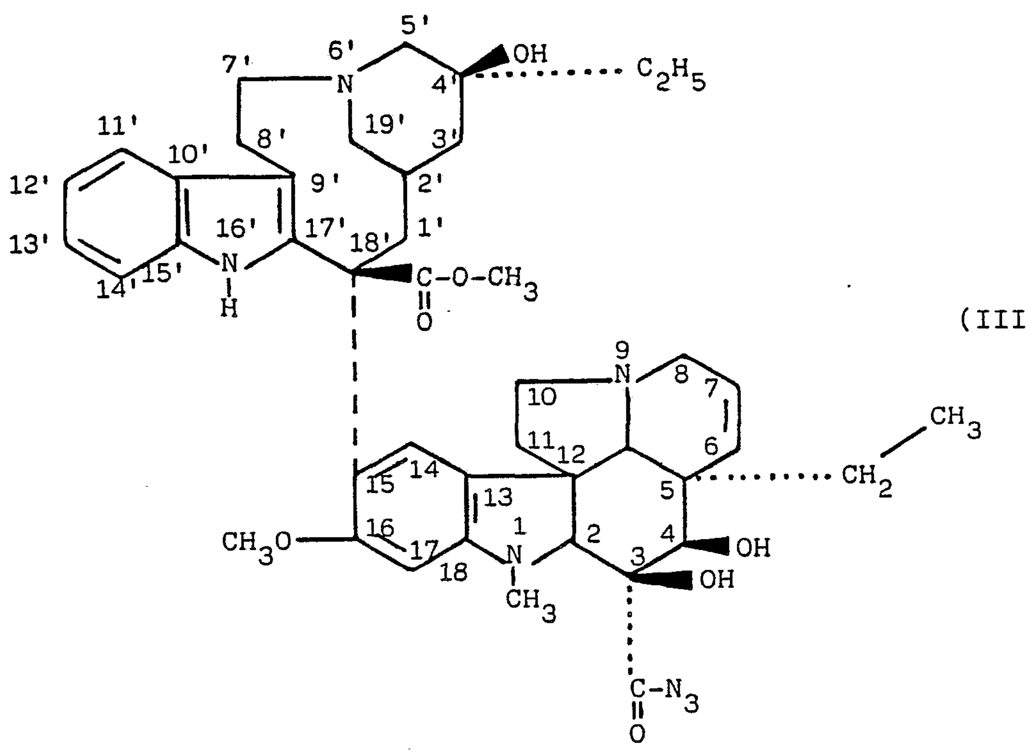

(III)

5. A pharmaceutical formulation comprising a conjugate according to any of claims 1 to 3 together with an parmaceutically-acceptable carrier or diluent therefor.

**Claims for the Contracting State: AT**

1. A process for producing an conjugate comprising an immunoglobulin or immunoglobulin fragment modified by one or more groups of the following formula which are covalently linked to it:

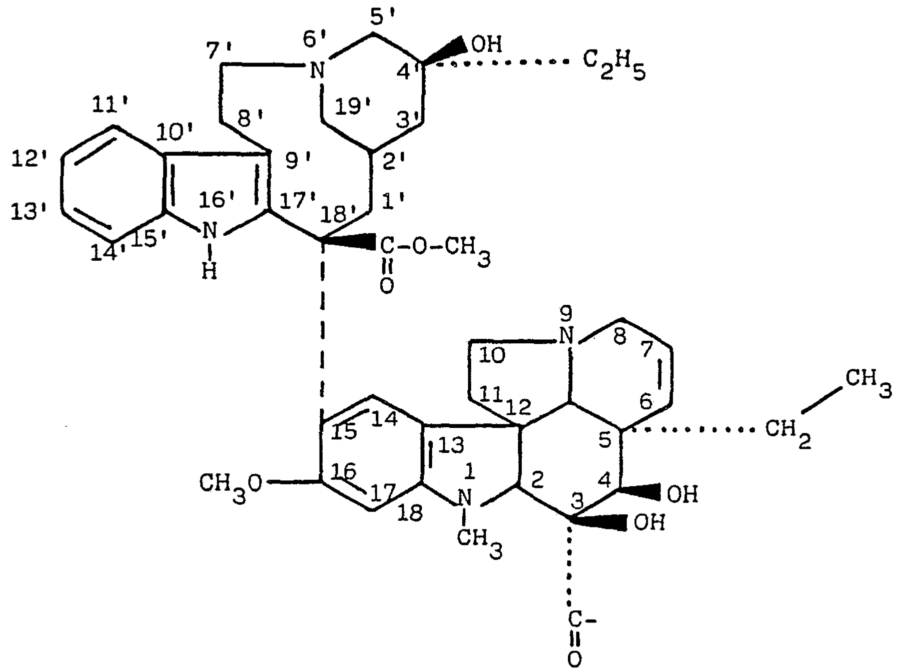

(II)

which comprises reacting an immunoglobulin or immunoglobulin fragment with desacetylvinblastine acid azide.

2. A process according to claim 1 for the preparation of a conjugate comprising an IgG immunoglobulin.

3. A process according to either of claims 1 and 2 in which the immunoglobulin or immunoglobulin fragment is modified by an average of from 2 to 5 groups of formula (II).

**Ansprüche für die Vertragsstaaten: BE CH DE FR GB IT LI LU NL SE**

1. Konjugat, enthaltend ein Immunoglobulin oder Immunoglobulinfragment, modifiziert durch eine oder mehrere Gruppen der nachstehenden Formel, welche kovalent daran gebunden sind:

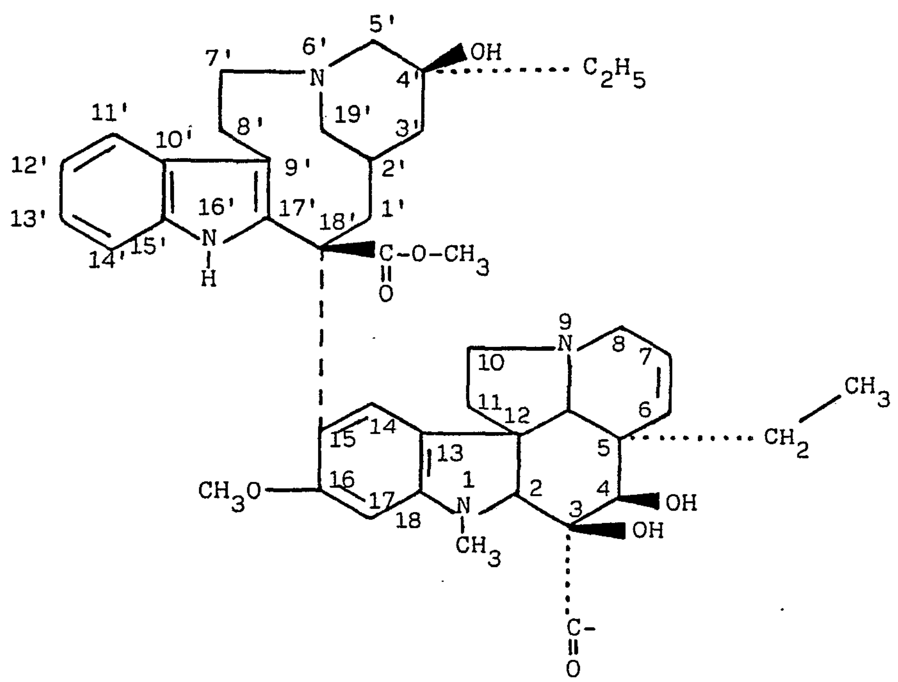

(II)

2. Konjugat nach Anspruch 1 unter Verwendung eines IgG-Immunoglobulins.

3. Konugat nach einem der Ansprüche 1 und 2, worin das Immunoglobulin oder Immunoglobulinfragment durch im Mittel 2 bis 5 Gruppen der Formel (II) modifiziert ist.

4. Verfahren zur Herstellung eines Konjugats nach Anspurch 1, welches umfaßf die Reaktion eines Immunoglobulins oder Immunoglobulinfragments mit einer Azidverbindung der Formel

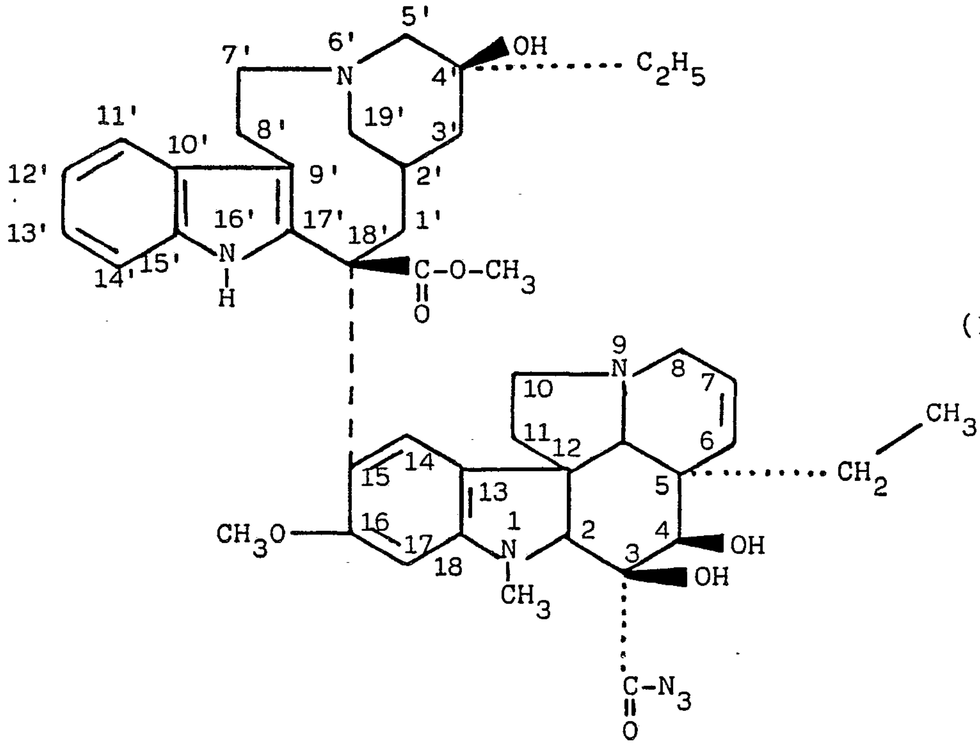

(III)

5. Pharmazeutische Formulierung, enthaltend ein Konjugat nach einem der Ansprüche 1 bis 3 zusammen mit einem pharmazeutisch annehmbaren Träger oder Verdünnungsmittel dafür.

**Ansprüche für den Vertragsstaat: AT**

1. Verfahren zur Herstellung eines Konjugats enthaltend ein Immunoglobulin oder Immunoglobulinfragment, modifiziert durch eine oder mehrere Gruppen der nachstehenden Formel, welche kovalent daran gebunden sind:

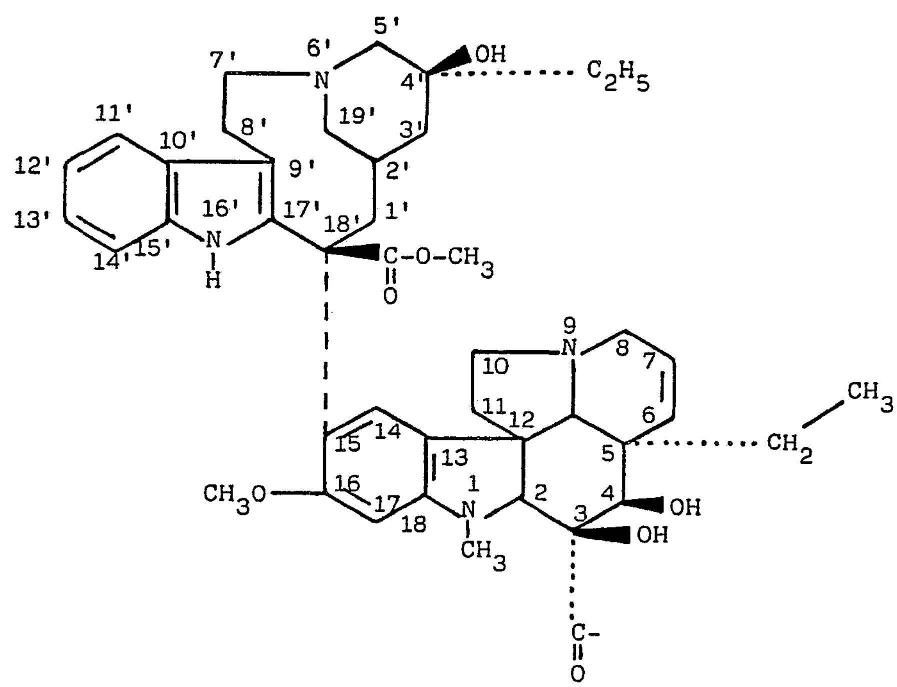

(II)

welches die Reaktion eines Immunoglobulins oder Immunoglobulinfragment mit Desacet ylvinblastinsäureazid umfaßt.

2. Verfahren nach Anspruch 1 zur Herstellung eines Konjugats, welches ein IgG-Immunoglobulin enthält.

3. Verfahren nach einem der Ansprüche 1 und 2, in welchem des Immunoglobulin oder Immunoglobulinfragment durch im Mittel 2 bis 5 Gruppen der Formel (II) modifiziert ist.

**Revendications pour les Etats contractants: BE CH DE FR GB IT LI LU NL SE**

1. Produit conjugué comprenant une immunoglobuline ou un fragment d'immunoglobuline modifié par un ou plusieurs groupes répondant à la formule suivante et qui y est ou y sont relié(s) par des liaisons covalentes:

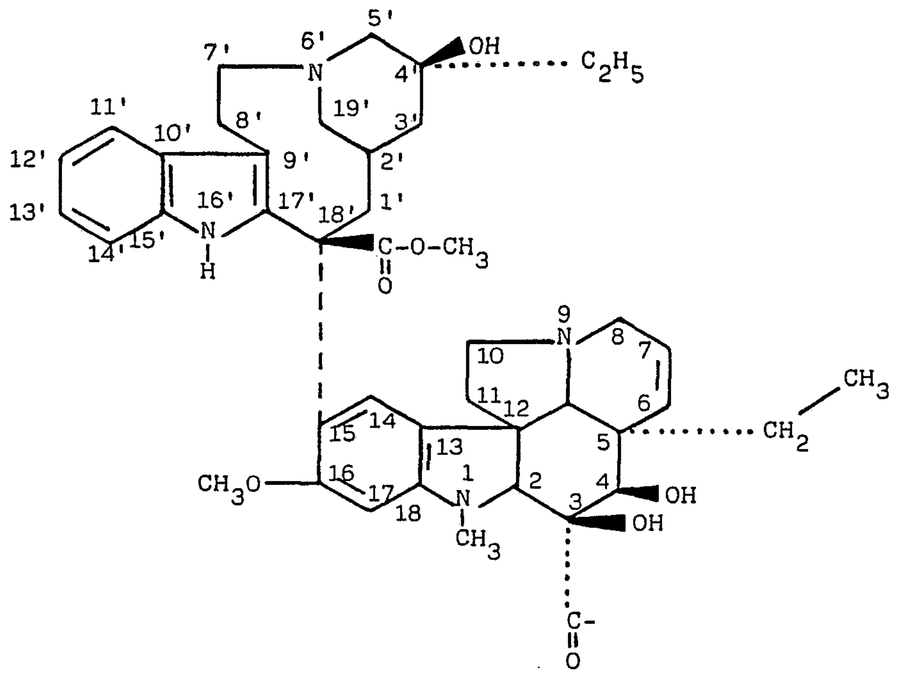

(II)

2. Produit conjugué selon la revendication 1, dans lequel on utilise une immunoglobuline IgG.

3. produit conjugé selon l'une quelconque des revendications 1 et 2, dans lequel l'immunoglobuline ou le fragment d'immunoglobuline est modifié par une moyenne de 2 à 5 groupes de formule (II).

4. Procédé de préparation d'un produit conjugué selon la revendication 1, caractérisé en ce qu'il consiste à faire réagir une immunoglobuline ou un fragment d'immunoglobuline avec un composé azide de formule:

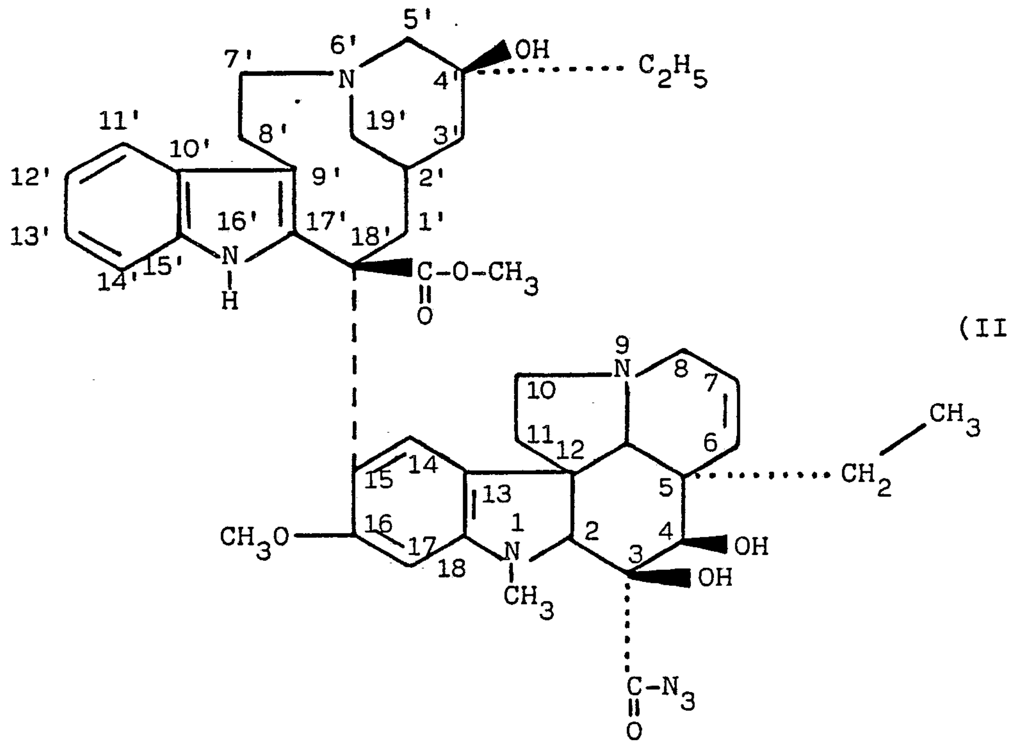

(III)

5. Formulation pharmaceutique comprenant un produit conjugé selong l'une quelconque des revendications 1 à 3, conjointement avec un diluant ou un support pharmaceutiquement acceptable pour ce produit conjugué.

**Revendications pour l'Etat contractant: AT**

1. Procédé de préparation d'un produit conjugué comprenant une immunoglobuline ou un fragment d'immunoglobuline modifié par un ou plusieurs groupes répondant à la formule suivante et qui y est ou y sont relié(s) par des liasions covalentes:

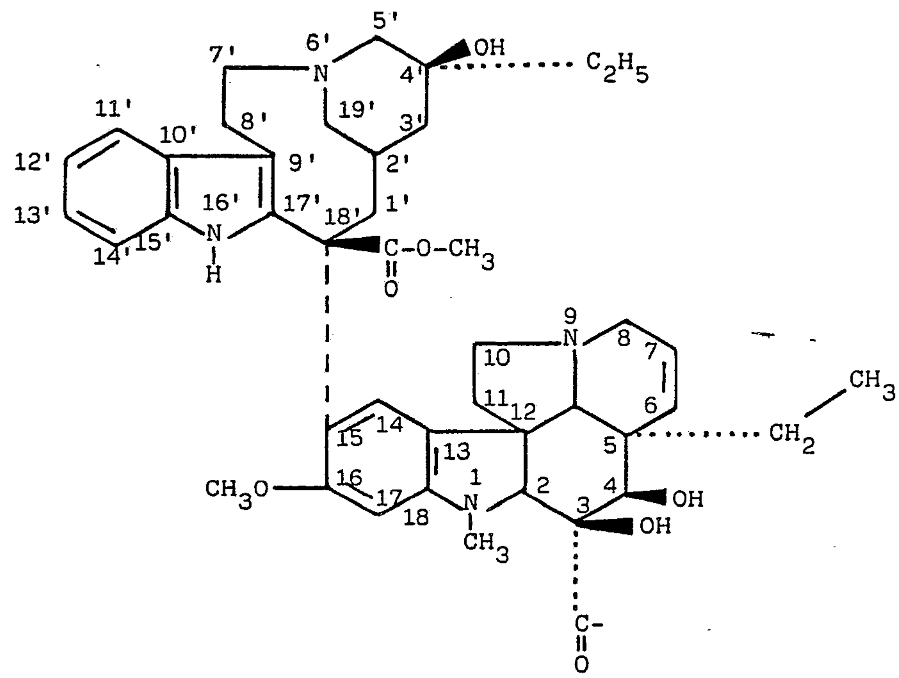

(II)

caractérisé en ce qu'il consiste à faire réagir une immunoglobuline ou un framgent d'immunoglobuline avec un azide d'acide de désacétylvinblastine.

2. Procédé selon la revendication 1 pour la préparation d'un produit conjugué comprenant une immunoglobuline IgG.

3. Procédé selon l'une quelconque des revendications 1 et 2, caractérisé en ce que l'immunoglobuline ou le fragment d'immunoglobuline est modifié par une moyenne de 2 à 5 groupes de formule (II).